(19) Europäisches Patentamt
European Patent Office
Office européen des brevets

(11) **EP 2 998 729 A1**

(12) **DEMANDE DE BREVET EUROPEEN**

(43) Date de publication:
**23.03.2016 Bulletin 2016/12**

(51) Int Cl.:
**G01N 23/04** *(2006.01)* **A61B 6/00** *(2006.01)*
**G06T 11/00** *(2006.01)*

(21) Numéro de dépôt: **15185175.5**

(22) Date de dépôt: **15.09.2015**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(30) Priorité: **16.09.2014 FR 1458693**

(71) Demandeur: **Commissariat à l'Énergie Atomique et aux Énergies Alternatives**
**75015 Paris (FR)**

(72) Inventeurs:
• **BLEUET, Pierre**
  **38180 SEYSSINS (FR)**
• **LALOUM, David**
  **38400 SAINT MARTIN D'HERES (FR)**

(74) Mandataire: **Brevalex**
**95, rue d'Amsterdam**
**75378 Paris Cedex 8 (FR)**

(54) **METHODE DE CORRECTION D'ARTEFACTS DANS DES IMAGES OBTENUES PAR TOMOGRAPHIE**

(57)　Méthode de correction d'artefacts que présente au moins une image tomographique d'un objet obtenue par un système de tomographie, ledit système de tomographie comprenant une source de rayons X polychromatique, un porte-échantillon et un détecteur, la position relative de la source de rayons X et du porte-échantillon étant modifiable de sorte à pouvoir modifier l'angle d'incidence entre les rayons X et l'objet, ledit objet comportant au moins deux matériaux, ladite méthode prenant en compte la variation d'un coefficient d'atténuation en fonction de l'énergie de la source de rayons X dans l'énergie du spectre de rayons X émis par la source de rayons X pour chacun des matériaux et ladite méthode prenant en compte l'existence d'au moins une discontinuité de la variation du coefficient d'atténuation d'au moins l'un des matériaux pour corriger les données acquises par le système de tomographie et reconstruire une image corrigée.

**EP 2 998 729 A1**

**Description**

## DOMAINE TECHNIQUE ET ÉTAT DE LA TECHNIQUE ANTÉRIEURE

La présente invention se rapporte à une méthode de correction d'artefacts dans des images obtenues par un procédé de caractérisation par tomographie.

L'intégration 3D est une voie prometteuse pour améliorer la performance des circuits intégrés. Cette technologie consiste en l'empilement de puces électroniques dans le but d'intégrer de nombreuses fonctionnalités (Processeurs, mémoires, MEMS, ...) dans un même dispositif. Les dispositifs ainsi réalisés sont plus fiables, plus performants et consomment moins d'énergie.

Les puces sont reliées entre elles par des interconnexions métalliques de quelques micromètres de diamètre, qui permettent la communication des puces entre elles, on cherche donc à réaliser des interconnexions fiables. Pour cela, on souhaite caractériser les interconnexions et plus généralement les structures ainsi formées.

Les moyens classiques de caractérisation tels que la microscopie électronique, la microscopie optique ou bien les faisceaux d'ions ne permettent d'analyser que la surface d'un échantillon. La tomographie par rayons X permet en revanche de caractériser les défauts internes d'un objet de façon non destructive. En effet, les rayons X sont bien plus pénétrants et permettent d'analyser de larges volumes de matière, à une résolution toutefois plus grossière.

Le principe de la tomographie par rayons X est le suivant: un faisceau d'électrons généré par un canon à électrons est concentré sur une cible métallique. Les électrons interagissent avec la cible métallique qui génère à son tour des rayons X.

Ceux-ci proviennent à la fois du rayonnement continu de freinage, dit « Bremsstrahlung », mais aussi du rayonnement caractéristique du matériau, dit rayonnement de fluorescence. Cet ensemble canon à électron - cible métallique est une source de rayons X ponctuelle.

Une partie de ce rayonnement irradie alors l'objet à caractériser, qui se trouve sur un porte-échantillon à 6 degrés de liberté : les 3 axes (x,y,z) et les 3 angles d'Euler.

Les photons non absorbés par l'échantillon sont alors collectés par une caméra CCD : on obtient une radiographie.

Le système de tomographie est basé sur l'acquisition d'une série de radiographies de l'objet obtenues par le détecteur tandis que le porte échantillon tourne sur lui-même sur un débattement angulaire de 180° plus l'angle du cône du faisceau de rayons X. Des algorithmes de reconstruction permettent alors d'obtenir une visualisation 3D de l'objet.

Des artéfacts de reconstruction peuvent apparaître en tomographie et peuvent dissimuler les défauts internes d'un échantillon. Ceux-ci sont bien connus de l'homme de métier et sont par exemple décrits dans le document J. Bahnart, « Advanced Tomographic Methods in Materials Reasearch and engineering », p. 145 149, 2008. Un de ces artefacts peut se traduire par des bandes sombres entre les interconnexions dans les images tomographiques lorsque plusieurs interconnexions sont scannées avec un faisceau de rayons X polychromatique.

De manière connue la présence de cet artefact est due au fait que les algorithmes de reconstruction classiques tels que la rétroprojection filtrée, les algorithmes de reconstruction algébriques considèrent le faisceau de rayons X comme monochromatique et que ce rayonnement est atténué par la loi simplifiée de Beer-Lambert.

$$I = I_0 . \exp(-\int_L \mu(l)dl)$$

Toutefois, étant donné que le faisceau considéré est polychromatique, lorsque celui-ci pénètre un échantillon, les photons de plus faible énergie sont absorbés plus facilement que les photons de haute énergie. Les valeurs des pixels sur les projections ne suivent alors pas la loi exponentielle décroissante observée avec l'équation ci-dessus. Ce phénomène peut générer des bandes sombres sur les images tomographiques lorsque plusieurs interconnexions sont scannées.

Ce phénomène est connu et de nombreuses méthodes existent pour corriger ces artéfacts de reconstruction, celles-ci sont par exemple décrites dans le document Van de Casteele et al., « A model-based correction method for beam hardening in X-ray microtomography » J X-ray Sci Technol, 12 (1) (2004), pp. 43-57*).

Cependant des artefacts ne sont pas corrigés par ces méthodes visant à traiter l'absorption des photons de basse énergie.

## EXPOSÉ DE L'INVENTION

C'est par conséquent un but de la présente invention d'offrir une méthode de correction des artefacts qui ne sont pas dus à l'absorption des photons de basse énergie.

Les inventeurs ont découvert que des artefacts apparaissaient lorsque les échantillons caractérisés par tomographie comportaient au moins un matériau dont le coefficient d'absorption ou d'atténuation en $\mu m^{-1}$ présentait une discontinuité, plus particulièrement il présentait un pic d'absorption ou d'atténuation. En effet, chaque matériau présente un coefficient d'atténuation qui diminue avec l'énergie des photons. Certains matériaux présentent une discontinuité dans leurs coefficients d'absorption. Par exemple, le cuivre présente une discontinuité dans ses coefficients d'absorption autour de 8,9 keV. Cette discontinuité se traduit par exemple par une brusque augmentation du coefficient d'absorption formant un pic sur la courbe de variation du coefficient d'absorption en fonction de l'énergie des photons.

Les inventeurs proposent alors une méthode de correction des artefacts qui prend en compte les discontinuités des coefficients d'absorption des matériaux analysés afin d'améliorer la qualité de la reconstruction.

Le but de la présente invention est alors atteint par une méthode de correction des artefacts lors de la caractérisation par tomographie à rayons X d'échantillons comportant au moins un matériau présentant une discontinuité dans ses coefficients d'atténuation, qui tient compte de cette discontinuité corriger les valeurs mesurées et à générer une image corrigée de laquelle le ou les artefacts ont disparu.

La méthode de correction peut comporter une étape de modification de la courbe de variation du coefficient d'atténuation en fonction de l'énergie des photons visant à au moins atténuer l'effet de la discontinuité et une étape de calcul d'un coefficient correcteur permettant de corriger les valeurs mesurées et à générer une image corrigée de laquelle le ou les artefacts ont disparu.

La méthode consiste par exemple à extrapoler les valeurs des coefficients d'absorption dans la zone du pic et à calculer le spectre qu'aurait mesuré le détecteur de photons en l'absence du pic. Ensuite un coefficient de correction est calculé à partir du spectre corrigé et du spectre non corrigé et une image est reconstruite en corrigeant les signaux mesurés par le détecteur.

La méthode de correction peut prévoir à partir de l'image tomographique initiale comportant les artefacts de réaliser des cartes d'épaisseur pour chacun des matériaux composant l'objet afin de tenir compte du coefficient d'atténuation de chaque matériau dans le calcul de l'absorption du spectre mesuré par le détecteur sans objet. Ce calcul est effectué avec les valeurs réelles des coefficients d'atténuation, ceux présentant une discontinuité et ceux ne présentant pas une discontinuité, et avec des valeurs corrigées des coefficients d'atténuation présentant la discontinuité. La connaissance de ces spectres permet de calculer le coefficient de correction.

De préférence les données prises en compte pour l'absorption du spectre mesuré par le détecteur sans objet avec les coefficients d'atténuation réels et/ou corrigés sont celles des pixels et angles d'incidence pour lesquels le rayonnement X a traversé un matériau présentant une discontinuité d'atténuation.

La présente invention a alors pour objet une méthode de correction d'artefacts que présente au moins une image tomographique d'un objet obtenue par un système de tomographie à rayons X, ledit système de tomographie comprenant une source de rayons X polychromatique, un porte-échantillon et un détecteur, la position relative de la source de rayons X et du porte-échantillon étant modifiable de sorte à pouvoir modifier l'angle d'incidence entre les rayons X et l'objet, ledit objet comportant au moins deux matériaux, ladite méthode prenant en compte la variation d'un coefficient d'atténuation en fonction de l'énergie de la source de rayons X dans l'énergie du spectre de rayons X émis par la source de rayons X pour chacun des matériaux et ladite méthode prenant en compte l'existence d'au moins une discontinuité de la variation du coefficient d'atténuation d'au moins l'un des matériaux pour corriger les données acquises par le système de tomographie et reconstruire une image corrigée.

De préférence, la prise en compte de l'existence d'au moins une discontinuité se traduit par une extrapolation de la variation du coefficient d'atténuation en fonction de l'énergie de la source de rayons X dans la zone de la discontinuité de sorte à atténuer la discontinuité.

La méthode de correction peu comporter les étapes suivantes en considérant que le détecteur comporte n pixels:

a) Acquisition de signaux par chaque pixel du détecteur pour différents angles d'incidence permettant la génération d'un sinogramme,
b) obtention d'une image tomographique à partir du sinogramme,
c) segmentation de l'image tomographique par matériau composant l'objet de sorte à obtenir autant d'images que de matériaux,
d) obtention d'un sinogramme pour chaque image obtenue à l'étape c),
e) obtention de cartes d'épaisseur pour chacun des matériaux à partir des sinogrammes de l'étape d),
f) détermination du spectre détecté par le détecteur en l'absence d'objet,
g) détermination d'un spectre, dit non corrigé, résultant de l'absorption du spectre détecté par les pixels du détecteur en l'absence d'objet par chacun des matériaux à tous les angles d'incidence et pour au moins une partie des pixels du détecteur à partir des cartes d'épaisseur en tenant compte des coefficients d'atténuation réels,
h) détection du ou des matériaux présentant une discontinuité de leur coefficient d'atténuation,
i) extrapolation des valeurs du coefficient d'atténuation pour chacun des matériaux présentant la discontinuité dans la zone de la discontinuité de sorte à atténuer la discontinuité, générant ainsi des valeurs de coefficient d'atténuation corrigés,

j) détermination d'un spectre, dit corrigé, résultant de l'absorption du spectre détecté par les pixels du détecteur en l'absence d'objet par chacun des matériaux à tous les angles d'incidence pour au moins une partie des pixels du détecteur à partir des cartes d'épaisseur en tenant compte des coefficients d'atténuation corrigés,

k) calcul du facteur de correction à partir des spectres corrigé et non corrigé,

l) génération d'un sinogramme corrigé en prenant en compte le facteur de correction pour corriger les données du sinogramme de l'étape a),

m) reconstruction d'une image tomographique à partir du sinogramme corrigé.

Le coefficient de correction est par exemple égal au rapport entre l'intégrale du spectre corrigé et l'intégrale du spectre non corrigé.

De préférence, lors de la détermination de spectre non corrigé, seuls sont pris en compte les couples pixel, angle d'incidence correspondant à une zone des cartes d'épaisseur comportant un matériau dont le coefficient d'atténuation présente une discontinuité.

De préférence également, lors de la détermination de spectre corrigé, seuls sont pris en compte les couples pixel, angle d'incidence correspondant à une zone des cartes d'épaisseur comportant un matériau dont le coefficient d'atténuation présente une discontinuité.

L'étape de détermination du spectre détecté par le détecteur en l'absence d'objets, dans le cas d'un détecteur direct, peut comporter les sous-étapes :

- détermination d'un temps d'acquisition donné tel que la probabilité qu'un pixel du détecteur reçoive deux photons durant ce temps d'acquisition est très faible,
- acquisition de radiographies en l'absence d'objets, le temps d'acquisition de chaque radiographie étant inférieur au temps d'acquisition donné,
- détermination de l'énergie reçue par chaque pixel,
- détermination du spectre détecté par le détecteur en l'absence d'objet.

Le temps d'acquisition est préférentiellement compris entre 10 ns et 1s.

La discontinuité dans les coefficients d'atténuation peut être un pic et la portion de courbe extrapolée peut s'étendre du début du pic dans le sens de l'énergie du spectre croissante à un point dont la valeur du coefficient d'atténuation est égale à celle du coefficient d'atténuation au début du pic augmenté par exemple de 10 % à 60 % de la hauteur du pic, de préférence de 20% à 50% de la hauteur du pic.

L'extrapolation est par exemple une extrapolation linéaire, une extrapolation logarithmique, une extrapolation exponentielle, une extrapolation polynomiale, spline...

**BRÈVE DESCRIPTION DES DESSINS**

La présente invention sera mieux comprise sur la base de la description qui va suivre et des dessins en annexe sur lesquels:

- la figure 1 est une représentation schématique d'un système de caractérisation par tomographie à rayons X pouvant être utilisé pour produire des images dont on souhaite corriger les artefacts par la méthode selon l'invention,
- la figure 2 est une représentation de la variation du coefficient d'atténuation du cuivre en $\mu m^{-1}$ en fonction de l'énergie des photons en eV et la correction apportée par la méthode selon l'invention,
- la figure 3 est une représentation de la variation du coefficient d'atténuation du nickel en $\mu m^{-1}$ en fonction de l'énergie des photons en eV,
- la figure 4 est une représentation de la variation du coefficient d'atténuation du zirconium en $\mu m^{-1}$ en fonction de l'énergie des photons en eV,
- la figure 5 est une représentation de la variation du coefficient d'atténuation du germanium en $\mu m^{-1}$ en fonction de l'énergie des photons en eV,
- la figure 6 est une image obtenue par tomographie à rayons X d'un échantillon de silicium comportant des interconnexions en cuivre avant correction,
- les figures 7A et 7B sont des images obtenues à partir de la segmentation de l'image de la figure 6 conformément au cuivre et au silicium respectivement,
- les figures 8A et 8B sont les cartes d'épaisseur obtenues à partir des images 7A et 7B respectivement,
- la figure 9 est une image tomographique de l'échantillon après correction par la méthode selon l'invention,
- la figure 10 est un organigramme de la méthode de correction selon l'invention,

- la figure 11 est un organigramme d'un exemple d'étape de détermination du spectre détecté par le détecteur de photons en l'absence d'échantillon,
- les figures 12 et 13 sont des représentations des spectres émis par différentes épaisseurs de cuivre avec les coefficients d'atténuation réels et les coefficients d'atténuation corrigées respectivement.

## EXPOSÉ DÉTAILLÉ DE MODES DE RÉALISATION PARTICULIERS

Dans la description qui va suivre les expressions "coefficient d'absorption" et "coefficient d'atténuation" sont synonymes et sont utilisées indifféremment.

La description détaillée de la méthode de correction utilise un échantillon comportant deux matériaux dont un présente une discontinuité de son coefficient d'atténuation, mais la présente invention s'applique également à la caractérisation d'objets comprenant au moins deux matériaux dont les deux matériaux présentent une discontinuité, et également à la caractérisation d'objets comportant au moins trois matériaux, au moins un des matériaux présentant une discontinuité de leur coefficient d'atténuation

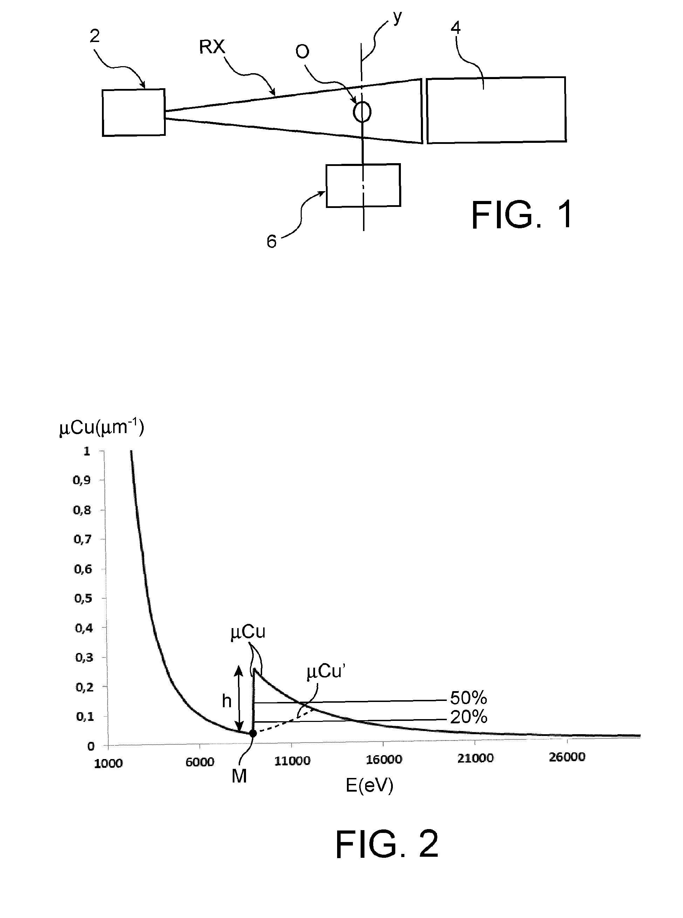

Sur la figure 1, on peut voir une représentation schématique d'un exemple d'un système de caractérisation par tomographie dont les images obtenues peuvent être corrigées par la méthode de correction selon l'invention.

Le système comporte une source 2 de rayons X, un détecteur de photons 4 et un porte-échantillon 6 disposé entre la source 2 et le détecteur 4 de sorte que, lorsqu'un objet O est monté sur le porte-échantillon 6, il soit éclairé par le faisceau de rayons X émis par la source C. Les photons non absorbés par l'échantillon sont collectés par le détecteur. Le détecteur comporte plusieurs pixels qui reçoivent chacun les photons et émettent un signal en fonction de l'énergie des photons reçue.

La source de rayons X est une source polychromatique.

Le porte-échantillon met en rotation l'échantillon sur lui-même autour d'un axe, par exemple un axe Y par rapport à la source. L'axe de rotation est vertical dans la représentation de la figure 1.

Le détecteur de photons 4 peut être une caméra CCD (Charge-Coupled Device en terminologie anglo-saxonne).

Le détecteur de photons 4 peut être à détection directe ou à détection indirecte, dans ce dernier cas le détecteur comporte un scintillateur convertissant le rayonnement visible émis par l'échantillon en électrons.

L'échantillon disposé sur le porte échantillon 6 est irradié par une partie du rayonnement émis par la source de rayons X, les photons non absorbés sont alors collectés par le détecteur et on obtient un radiographie. Le porte-échantillon pivote entre chaque radiographie pour modifier l'angle entre l'échantillon et le faisceau. Ensuite par reconstruction, on obtient une image en trois dimensions de l'échantillon.

Dans la description qui va suivre, l'objet à caractériser est un objet bi-matériaux composé de plusieurs régions à fort numéro atomique (Z) intégré dans un matériau à bas Z. Le matériau à haut Z a un pic d'absorption dans l'énergie du spectre de rayons X émis par la source.

Dans l'exemple choisi, le matériau à haut numéro atomique est le cuivre et le matériau à bas numéro atomique est le silicium. Par exemple l'objet comporte un substrat en silicium et des interconnexions en cuivre, ces interconnexions peuvent servir à l'interconnexion 3D des circuits intégrés. Les interconnexions s'étendent par exemple perpendiculairement au plan du substrat.

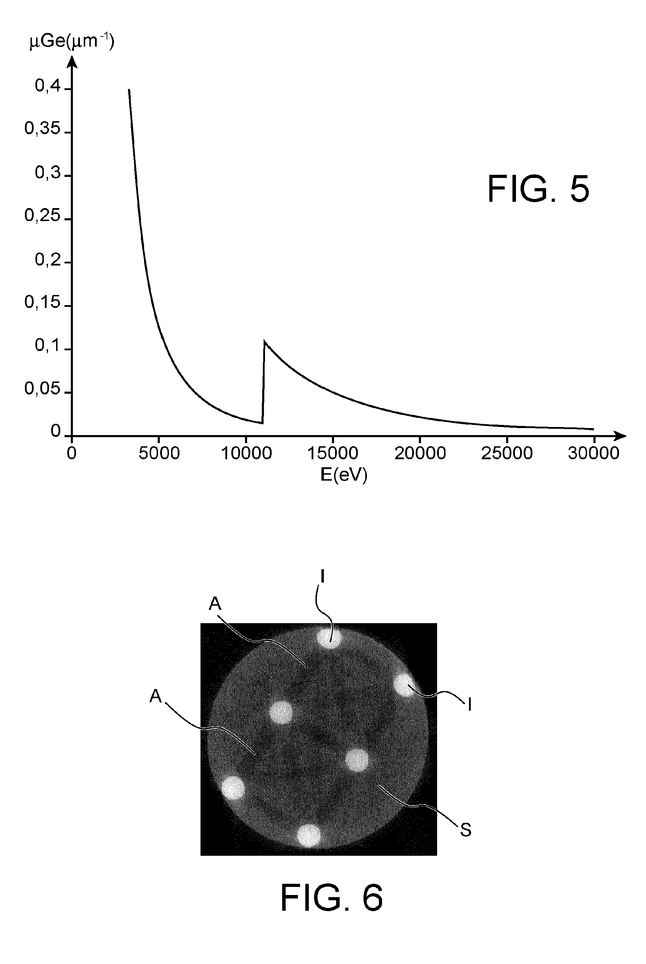

Sur la figure 6, on peut voir une image tomographique de l'échantillon obtenue sans que la méthode de correction selon l'invention lui soit appliquée. Le substrat est désigné par S et les interconnexions par I. On constate la présence d'artefacts A formant des bandes sombres entre les interconnections I. Les inventeurs ont découvert que ces bandes sombres A étaient dues à l'utilisation d'un faisceau de rayons X polychromatique et à l'existence d'une discontinuité dans le coefficient d'atténuation du cuivre.

Sur la figure 2, on peut voir la variation réelle de la valeur du coefficient d'atténuation du cuivre en $\mu m^{-1}$ en fonction de l'énergie des photons en eV. On constate que vers 8900 eV, ce coefficient présente un pic. Les inventeurs ont déterminé que les photons dont l'énergie était au-dessus du pic étaient préférentiellement absorbés, provoquant l'apparition des bandes sombres sur l'image tomographique.

Par exemple, la méthode de correction vise à générer une nouvelle courbe de variation du coefficient d'absorption du cuivre sur laquelle la discontinuité est atténuée, voire supprimée, en extrapolant les valeurs du coefficient d'atténuation au niveau de la discontinuité et en calculant un coefficient correcteur permettant de reconstruire une image tomographique sans bande sombre.

Dans la présente demande, on entend par "discontinuité" la brusque variation du coefficient d'atténuation telle que la courbe présente un pic.

Un exemple de la méthode de correction va maintenant être décrit. Nous allons tout d'abord définir les paramètres et variables utilisées. On définit :

- $R_{meas}(i,\phi)$ le sinogramme mesuré avec i le $i^{ème}$ pixel et $\phi$ l'angle de projection.
- $R_{corr}(i,\phi)$ le sinogramme corrigé.

- $R_{Cu}(i,\phi)$ le sinogramme issu de l'image segmentée avec uniquement la contribution du Cu
- $R_{Si}(i,\phi)$ le sinogramme issu de l'image segmentée avec uniquement la contribution du Si.
- $\mu_{Cu}(E)$ les coefficients d'absorption réels du Cu.
- $\mu_{Cu'}(E)$ les coefficients d'absorption modifiés du Cu.
- $\mu_{Si}(E)$ les coefficients d'absorption du Si.
- $\overline{I}_0(E)$ le spectre mesuré par le détecteur de photons X, i.e. l'intensité détectée en fonction de l'énergie du faisceau,
- lo(E) le spectre émis par la source de rayons X en l'absence d'objet, i.e. l'intensité détectée par le détecteur en l'absence d'objet,
- $S_p$ la taille d'un pixel du détecteur.
- $S_v$ la taille virtuelle du pixel du détecteur définie par $S_v = S_p/M$, dans le cas d'un faisceau conique, avec M le grandissement géométrique du système de tomographie; $S_v$ est définie par $S_v = S_p$ dans le cas d'un faisceau parallèle.

Nous rappelons qu'un sinogramme est le signal issu d'une projection de l'objet à analyser selon différents angles d'incidence $\phi$. On obtient alors une matrice R de taille I x $\phi$ avec I est le nombre de pixels du détecteur dans le plan de la coupe et $\phi$ le nombre d'angles de projection.

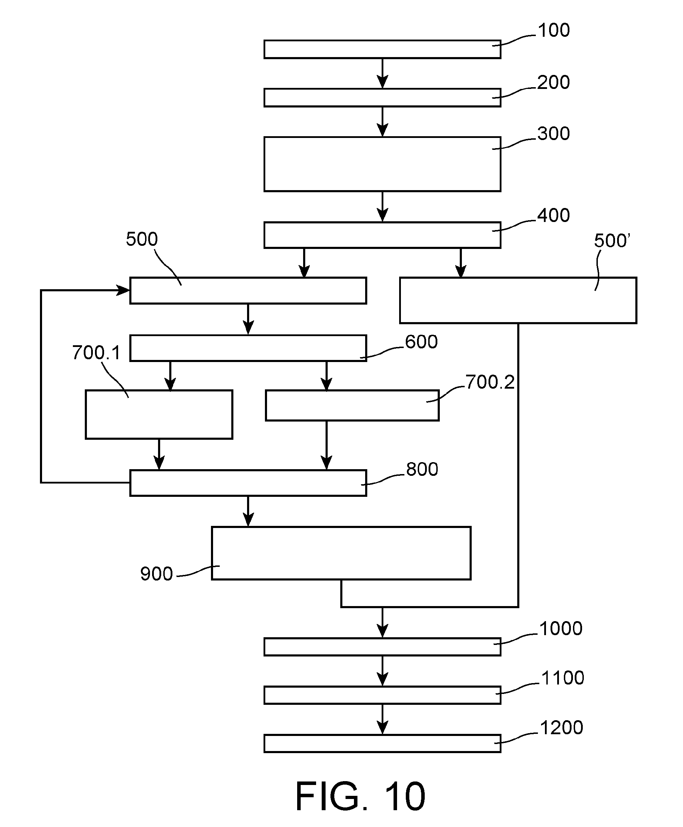

Sur la figure 10, on peut voir un organigramme schématisant les étapes d'un exemple de la méthode de correction selon la présente invention.

Lors d'une première étape 100, on dispose l'objet sur le porte-échantillon afin d'acquérir des mesures pour réaliser un sinogramme non corrigé $R_{meas}(i,\phi)$. Pour cela on acquiert les mesures fournies par les pixels du détecteur pour $\phi$ angles de rotation du porte-échantillon.

Lors d'une étape suivante 200, on reconstruit une première image tomographique à partir du sinogramme non corrigé, celle-ci est représentée sur la figure 6. Elle comporte des bandes sombres.

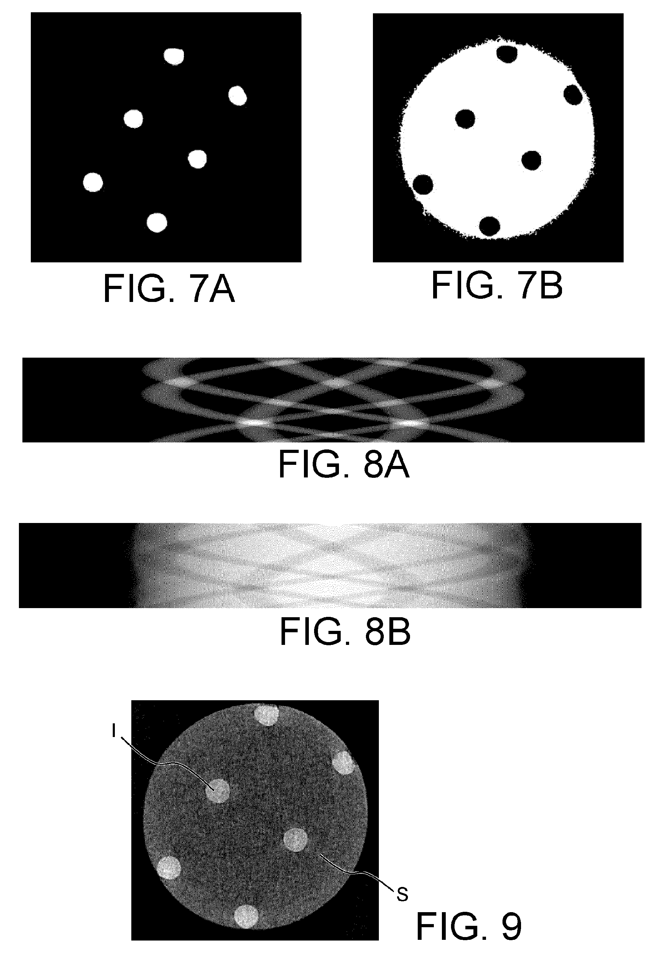

Lors d'une étape suivante 300, on effectue une segmentation de l'image tomographique obtenue à l'étape 100 en fonction du matériau. Pour la première image segmentée on attribue 1 au matériau à Z élevé, par exemple le cuivre, et 0 au matériau à bas Z, par exemple le silicium. Pour la deuxième image on attribue 0 au Cu et 1 au Si. On obtient les images des figures 7A et 7B respectivement, le Cu apparaissant en blanc sur la figure 7A et en noir sur la figure 7B et inversement pour le Si.

Dans le cas d'un objet comportant au moins trois matériaux, suite à l'étape de segmentation on obtient autant d'images que de matériaux composant l'objet, et pour chque image on attribue 1 à l'un des matériaux et 0 aux autres matériaux.

Ensuite, on réalise à partir de chacune des images segmentées un sinogramme en reprojetant les données des images segmentées conformément aux conditions d'acquisition, i.e. avec les angles selon lesquels les radiographies ont été prises.

Les sinogrammes sont alors normalisés en étant multipliés par la taille virtuelle du détecteur Sv afin d'obtenir deux cartes d'épaisseur de matériau traversé par le rayonnement X : $R_{Cu}(i,\phi) \times S_V$ (figure 8A) et $R_{Si}(i,\phi) \times S_V$ figure 8B). Dans la suite de la description, la taille virtuelle du détecteur Sv n'est pas reprise à des fins de simplicité. Les cartes d'épaisseur sont désignées également par $R_{Cu}(i,\phi)$ et $R_{Si}(i,\phi)$.

Lors d'une étape suivante 400, on détermine le spectre $\overline{I}_0(E)$ détecté par le détecteur.

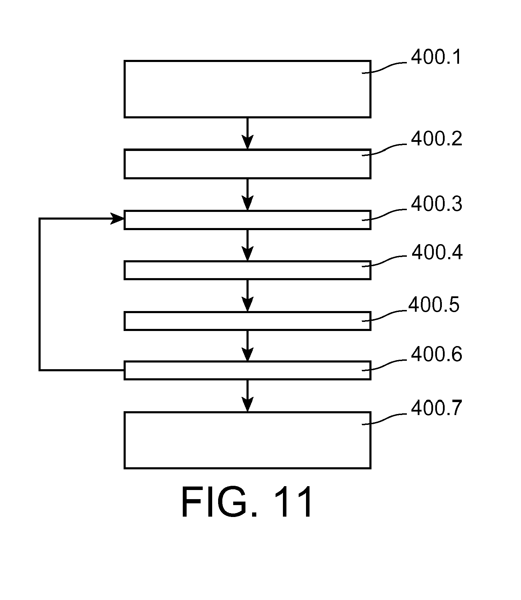

Un exemple de cette étape est détaillé sur l'organigramme de la figure 11.

Nous nous plaçons dans le cas d'un détecteur direct.

Lors d'une première étape 400.1, on détermine un temps d'acquisition tel que la probabilité qu'un même pixel du détecteur reçoive deux photons soit très faible, par exemple inférieure à $1/10^6$. Le temps d'acquisition est par exemple compris entre 10 ns et 1s.

Lors d'une étape suivante 400.2, on acquiert un grand nombre de radiographies sans échantillon, par exemple au moins une centaine, le temps d'acquisition de chacune des radiographies étant inférieure au temps d'acquisition déterminé à l'étape 400.1. Ce temps dépend de la brillance de la source. Ce temps est choisi suffisamment court pour rendre faible la probabilité qu'un même pixel du détecteur reçoive deux photons.

Chaque pixel retourne alors une valeur proportionnelle à l'énergie du photon incident.

Lors d'une étape suivante 400.3, on sélectionne une radiographie.

Lors d'une étape suivante 400.4, on applique à cette radiographie la loi de conversion ADUs (analog to digital units) - énergie du photon incident qui est connue pour chaque détecteur, celle-ci étant en général fournie par le constructeur du détecteur,

Lors d'une étape suivante 400.5, un histogramme est réalisé à partir des résultats de l'étape 400.4.

Tant qu'il reste des radiographies auxquelles la loi de conversion n'a pas été appliquée (étape 400.6) on répète les étapes 400.3, 400.4 et 400.5.

Lors d'une étape suivante 400.7, on moyenne les histogrammes obtenus ce qui fournit le spectre $\overline{I}_0(E)$ détecté par le détecteur.

Dans le cas d'un détecteur à détection indirecte, un scintillateur convertissant les photons X en photons visibles est prévu entre l'échantillon et le détecteur, ces photons visibles étant alors détectés par une caméra CCD. On simule le spectre I(E) généré par la source de rayons X, par exemple au moyen du logiciel de simulation Win X-ray. La conversion photons X - photons visibles via le scintillateur peut ensuite être simulée via le logiciel de simulation Sindbad. Le spectre $\overline{I}_0(E)$ détecté par le détecteur peut alors être obtenu.

Lors d'une étape suivante 500', à partir de la connaissance du spectre $\overline{I}_0(E)$, on simule l'absorption du spectre $\overline{I}_0(E)$ pour chaque pixel du sinogramme par les matériaux de l'échantillon en utilisant les cartes d'épaisseur de matériaux traversés $R_{Cu}(i,\phi)$ et $R_{Si}(i,\phi)$ obtenus à l'étape 300.

On définit $\overline{I}(i, \phi, E)$ l'ensemble des spectres résultants de l'absorption de $\overline{I}_0(E)$ par les matériaux présents dans l'échantillon. $\overline{I}(i, \phi, E)$ est calculé de la façon suivante :

$$\overline{I}(i,\Phi,E) = \overline{I}_0(E) \exp\left(-\mu_{Si}(E).R_{Si}(i,\Phi)\right) \exp\left(-\mu_{Cu}(E).R_{Cu}(i,\Phi)\right) \text{ (EQ1)}$$

L'équation (EQ1) ci-dessus est générale et fait calculer l'absorption de $\overline{I}_0(E)$ pour chaque point (i, $\phi$), i.e. pour chaque pixel et pour tous les angles d'incidence du sinogramme. Cette étape peut avantageusement être simplifiée en ne calculant l'absorption de $\overline{I}_0(E)$ que pour les points (i, $\phi$) où le rayonnement a traversé un matériau présentant un pic d'absorption, plus généralement une discontinuité d'absorption, ce qui permet de réduire le nombre de points.

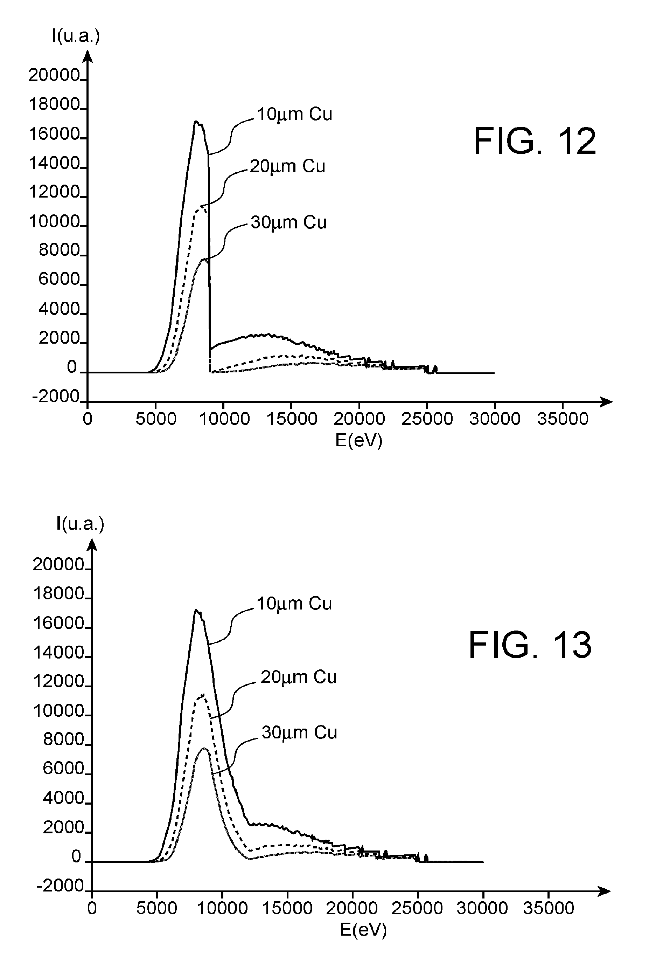

Sur la figure 12, on peut voir le spectre $\overline{I}(i, \phi, E)$ simulé pour une épaisseur de 60 $\mu$m de Si et 10 $\mu$m de Cu, 20 $\mu$m de Cu et 30 $\mu$m de Cu, i.e. le spectre $I_0$ après avoir traversé 60$\mu$m de Si et 10$\mu$m de Cu, $I_0$ après 60$\mu$m de Si et 20$\mu$m de Cu et $I_0$ après 60$\mu$m de Si et 30$\mu$m de Cu respectivement. La variation de l'épaisseur de Cu est relative à la modification de l'angle d'incidence du rayonnement X. $\overline{I}(i, \phi, E)$ est calculé en tenant compte de l'absorption de $I_0$ après toutes les combinaisons (épaisseur Si, épaisseur Cu) rencontrées.

On constate que l'intensité mesurée pour chacune des épaisseurs de cuivre au-delà de 8900 eV chute, les photons dont l'énergie est supérieure à 8900 eV sont effectivement préférentiellement absorbés comme l'ont constaté les inventeurs.

Par ailleurs, lors d'une étape 500 on sélectionne l'un des matériaux présent dans l'objet, par exemple le cuivre.

Dans une étape suivante 600, on détermine si ce matériau présente un pic d'absorption dans le spectre émis par la source de rayons X, ce qui est le cas du cuivre.

Alors, lors d'une étape 700.1, on limite l'effet du pic d'absorption du cuivre en extrapolant les valeurs des coefficients d'absorption $\mu_{Cu}(E)$. On atténue, avantageusement on supprime numériquement le pic d'absorption à l'origine de l'artéfact.

Par exemple, une méthode pour limiter l'effet du pic d'absorption débute l'extrapolation des coefficients d'absorption à partir du point M de la courbe où se trouve la discontinuité (figure 2), le point M marquant le début du pic dans le sens de l'énergie croissante.

Par exemple:

a) on mesure la hauteur h du pic d'absorption,
b) on supprime le pic en extrapolant les valeurs à partir du point M en récupérant la courbe réelle après le pic. De préférence on récupère la courbe dans un intervalle du coefficient d'atténuation compris entre 10% et 60%; de préférence entre 20 % et 50 % de la hauteur du pic comme cela est représenté sur la figure 2,
c) l'extrapolation des valeurs des coefficients d'absorption peut être réalisée par différentes solutions numériques, telles que l'extrapolation linéaire, l'extrapolation logarithmique, l'extrapolation exponentielle, l'extrapolation polynomiale, spline... dans l'exemple de la figure 2, la correction est obtenue par extrapolation exponentielle.

A la fin de l'étape 700.1, on obtient de nouveaux coefficients d'atténuation $\mu_{Cu'}(E)$ au niveau de la discontinuité.

Dans le cas où le matériau sélectionné ne présente pas de discontinuité de son coefficient d'absorption, ce qui est le cas du silicium, le coefficient d'absorption est inchangée $\mu_{j'}(E) = \mu_j(E)$ (étape 700.2). De manière générale, j est un entier positif supérieur ou égal à 1. Dans l'exemple décrit, j est égal à 1 puis égal à 2.

Ensuite lors d'une étape 800, on vérifie si l'objet comporte un autre matériau. Si oui on retourne à l'étape 500, ce qui est le cas de notre objet, il comporte également du silicium.

A l'étape 600, on constate que son coefficient d'absorption ne présente pas de discontinuité dans le spectre émis par la source de rayons X, on passe à l'étape 700.2, le coefficient d'absorption est inchangée $\mu_{Si'}(E) = \mu_{Si}(E)$ (étape 700.2).

Lorsque les nouveaux coefficients d'absorption pour tous les matériaux ont été déterminés (inchangés ou extrapolés), on réalise une simulation de l'absorption du spectre $\overline{I}_0(E)$ pour chaque pixel du sinogramme (étape 900)

Dans l'exemple pris en compte, l'objet n'ayant que deux matériaux, on passe à l'étape 900.

L'ensemble des spectres résultant de cette simulation est désigné par $\bar{I}'(i, \phi, E)$.

$\bar{I}'(i, \phi, E)$ est calculé de la façon suivante :

$$\bar{I}'(i, \Phi, E) = \bar{I}_0(E) \exp\left(-\mu_{Si}(E).R_{Si}(i, \Phi)\right) \exp\left(-\mu_{Cu'}(E).R_{Cu}(i, \Phi)\right)$$

Comme pour la détermination de $\bar{I}(i, \phi, E)$, cette étape peut être avantageusement simplifiée en ne calculant l'absorption de $I_0(E)$ que pour les points $(i, \phi)$ où le rayonnement a traversé un matériau présentant un pic d'absorption.

Sur la figure 13, on peut voir le spectre $\bar{I}'(i, \phi, E)$ simulé pour une épaisseur de 60 $\mu$m de Si et 10 $\mu$m de Cu, 20 $\mu$m de Cu et 30 $\mu$m de Cu.

On constate que, grâce à la réduction de la discontinuité, l'intensité calculée pour chacune des épaisseurs de cuivre au-delà de 8900 eV ne chute plus. La réduction de la discontinuité permet donc effectivement de pallier cette absorption.

Lors d'une étape suivante 1000, à partir de la connaissance de $\bar{I}'(i, \phi, E)$ déterminé à l'étape 900 et $\bar{I}(i, \phi, E)$ déterminé à l'étape 500', on calcule le rapport entre les intégrales des spectres modifiés $\bar{I}'(i, \phi, E)$ et les intégrales des spectres non modifiés $\bar{I}(i, \phi, E)$ en utilisant les équations (EQ1) et (EQ2). On obtient alors un coefficient correcteur $\alpha(i,\phi)$ défini par :

$$\alpha(i, \Phi) = \frac{\int \bar{I}'(i,\Phi,E)dE}{\int \bar{I}(i,\Phi,E)dE} \qquad (EQ3).$$

Lors d'une étape suivante 1100, on corrige le sinogramme mesuré à l'étape 100 en lui appliquant le coefficient correcteur donné par l'équation (EQ3). On obtient alors le sinogramme corrigé $R_{corr}(i,\phi)$, défini par :

$$R_{corr}(i, \Phi) = \alpha(i, \Phi).R_{meas}(i, \Phi)$$

A partir du sinogramme corrigé, on reconstruit l'image tomographique (étape 1200), l'image obtenue est représentée sur la figure 9. On constate que les bandes sombres n'apparaissent plus, la méthode de correction est donc efficace.

Dans le cas où l'objet à caractériser comporte plus de deux matériaux, i.e. j > 2, on applique la même méthode en généralisant les équations les équations EQ1 et EQ2 généralisées.

EQ1 s'écrit alors :

$$\bar{I}(i, \Phi, E) = \bar{I}_0(E) \prod_{j=1}^{n} \exp\left(-\mu_j(E).R_j(i, \Phi)\right)$$

Avec j le j<sup>ème</sup> matériau.

EQ2 s'écrit alors :

$$\bar{I}'(i, \Phi, E) = \bar{I}_0(E) \prod_{j=1}^{n} \exp\left(-\mu_{j'}(E).R_j(i, \Phi)\right)$$

On simule le spectre résultant de l'absorption du spectre $\bar{I}_0(E)$ par tous les matériaux formant l'objet avec les coefficients d'absorption non corrigés et celui résultant de l'absorption du spectre $\bar{I}_0(E)$ par tous les matériaux formant l'objet avec les coefficients d'absorption corrigés et on calcule le rapport des intégrales de chacun de des spectres, ce qui permet ensuite de corriger le sinogramme initial et de reconstruire une image tomographique sans artefact.

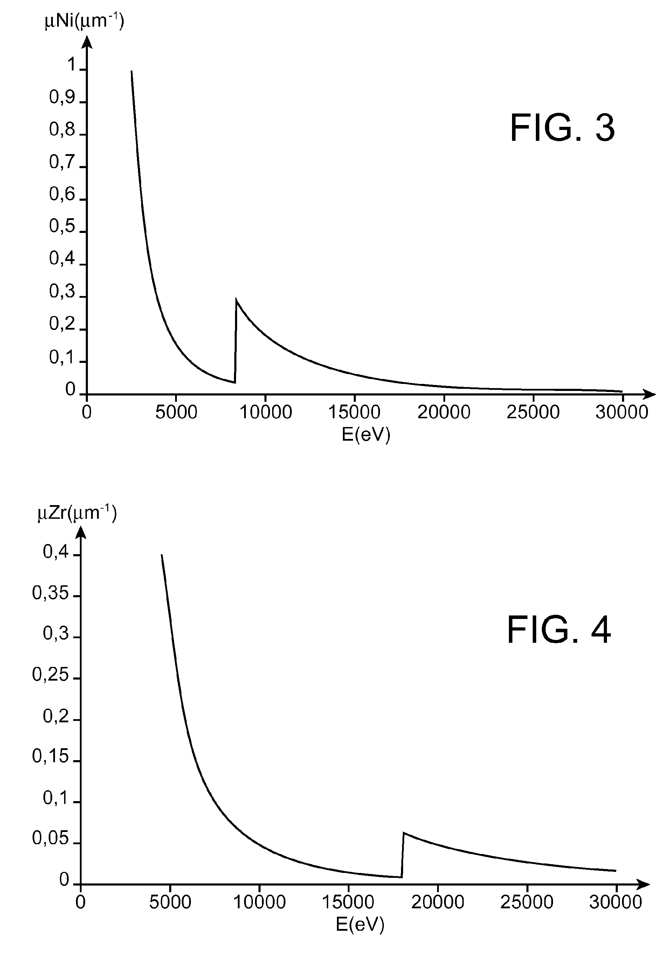

D'autres matériaux présentent une discontinuité dans leur coefficient d'absorption. Sur la figure 3, on peut voir la variation du coefficient d'atténuation du nickel en $\mu$m$^{-1}$ en fonction de l'énergie des photons en eV, on observe l'existence d'un pic d'absorption à 8,3 keV. Sur la figure 4, on peut voir une représentation de la variation du coefficient d'atténuation du zirconium en $\mu$m$^{-1}$ en fonction de l'énergie des photons en eV, on observe l'existence d'un pic d'absorption à 17,9 keV. La figure 5 représente la variation du coefficient d'atténuation du germanium en $\mu m^{-1}$ en fonction de l'énergie des photons en eV, on observe l'existence d'un pic d'absorption à 11 keV.

Dans le cas d'un objet comportant par exemple des éléments de zirconium, des éléments en germanium et des éléments en nickel dans un substrat en silicium, on pourrait appliquer la méthode de correction selon l'invention, en particulier les étapes des organigrammes 10 et 11 avec $j \leq 4$

La méthode de correction s'applique également à la correction d'un artefact résultant de matériaux présentant au moins deux discontinuités dans leurs coefficients d'absorption sur le spectre d'émission de la source de rayons X.

**Revendications**

1. Méthode de correction d'artefacts que présente au moins une image tomographique d'un objet obtenue par un système de tomographie à rayons X, ledit système de tomographie comprenant une source de rayons X polychromatique, un porte-échantillon et un détecteur, la position relative de la source de rayons X et du porte-échantillon étant modifiable de sorte à pouvoir modifier l'angle d'incidence entre les rayons X et l'objet, ledit objet comportant au moins deux matériaux, ladite méthode prenant en compte la variation d'un coefficient d'atténuation en fonction de l'énergie de la source de rayons X dans l'énergie du spectre de rayons X émis par la source de rayons X pour chacun des matériaux et ladite méthode prenant en compte l'existence d'au moins une discontinuité de la variation du coefficient d'atténuation d'au moins l'un des matériaux pour corriger les données acquises par le système de tomographie et reconstruire une image corrigée, ladite méthode de correction comportant une étape de modification d'une courbe de variation du coefficient d'atténuation en fonction de l'énergie des photons visant à au moins atténuer l'effet de la discontinuité et une étape de calcul d'un coefficient correcteur permettant de corriger les valeurs acquises et à générer une image corrigée de laquelle le ou les artefacts ont disparu.

2. Méthode de correction selon la revendication 1, dans laquelle la prise en compte de l'existence d'au moins une discontinuité se traduit par une extrapolation de la variation du coefficient d'atténuation en fonction de l'énergie de la source de rayons X dans la zone de la discontinuité de sorte à atténuer la discontinuité.

3. Méthode de correction selon la revendication 2, le détecteur comportant n pixels, ladite méthode comportant les étapes :

   a) acquisition de signaux par chaque pixel du détecteur pour différents angles d'incidence permettant la génération d'un sinogramme,
   b) obtention d'une image tomographique à partir du sinogramme,
   c) segmentation de l'image tomographique par matériau composant l'objet de sorte à obtenir autant d'images que de matériaux,
   d) obtention d'un sinogramme pour chaque image obtenue à l'étape c),
   e) obtention de cartes d'épaisseur pour chacun des matériaux à partir des sinogrammes de l'étape d),
   f) détermination du spectre détecté par le détecteur en l'absence d'objet,
   g) détermination d'un spectre, dit non corrigé, résultant de l'absorption du spectre détecté par les pixels du détecteur en l'absence d'objet par chacun des matériaux à tous les angles d'incidence et pour au moins une partie des pixels du détecteur à partir des cartes d'épaisseur en tenant compte des coefficients d'atténuation réels,
   h) détection du ou des matériaux présentant une discontinuité de leur coefficient d'atténuation,
   i) extrapolation des valeurs du coefficient d'atténuation pour chacun des matériaux présentant la discontinuité dans la zone de la discontinuité de sorte à atténuer la discontinuité, générant ainsi des valeurs de coefficient d'atténuation corrigés,
   j) détermination d'un spectre, dit corrigé, résultant de l'absorption du spectre détecté par les pixels du détecteur en l'absence d'objet par chacun des matériaux à tous les angles d'incidence pour au moins une partie des pixels du détecteur à partir des cartes d'épaisseur en tenant compte des coefficients d'atténuation corrigés,
   k) calcul du facteur de correction à partir des spectres corrigé et non corrigé,
   l) génération d'un sinogramme corrigé en prenant en compte le facteur de correction pour corriger les données du sinogramme de l'étape a),
   m) reconstruction d'une image tomographique à partir du sinogramme corrigé.

4. Méthode de correction selon la revendication 3, dans laquelle le coefficient de correction est égal au rapport entre l'intégrale du spectre corrigé et l'intégrale du spectre non corrigé.

**5.** Méthode de correction selon la revendication 3 ou 4, dans laquelle, lors de la détermination de spectre non corrigé, seuls sont pris en compte les couples pixel, angle d'incidence correspondant à une zone des cartes d'épaisseur comportant un matériau dont le coefficient d'atténuation présente une discontinuité.

**6.** Méthode de correction selon l'une des revendications 3 à 5, dans laquelle, lors de la détermination de spectre corrigé, seuls sont pris en compte les couples pixel, angle d'incidence correspondant à une zone des cartes d'épaisseur comportant un matériau dont le coefficient d'atténuation présente une discontinuité.

**7.** Méthode de correction selon l'une des revendications 3 à 6, dans laquelle l'étape de détermination du spectre détecté par le détecteur en l'absence d'objets, dans le cas d'un détecteur direct, présente les sous-étapes :

- détermination d'un temps d'acquisition donné tel que la probabilité qu'un pixel du détecteur reçoive deux photons durant ce temps d'acquisition est probabilité inférieure à $1/10^6$,
- acquisition de radiographies en l'absence d'objets, le temps d'acquisition de chaque radiographie étant inférieur au temps d'acquisition donné,
- détermination de l'énergie reçue par chaque pixel,
- détermination du spectre détecté par le détecteur en l'absence d'objet.

**8.** Méthode de correction selon la revendication 7, dans laquelle le temps d'acquisition est compris entre 10 ns et 1s.

**9.** Méthode de correction selon l'une des revendications 2 à 8, dans laquelle la discontinuité est un pic et la portion de courbe extrapolée s'étend du début du pic dans le sens de l'énergie du spectre croissante à un point dont la valeur du coefficient d'atténuation est égale à celle du coefficient d'atténuation au début du pic augmenté de 10 % à 60 % de la hauteur du pic, de préférence de 20% à 50% de la hauteur du pic.

**10.** Méthode de correction selon l'une des revendications 2 à 9, dans laquelle l'extrapolation est une extrapolation linéaire, une extrapolation logarithmique, une extrapolation exponentielle, une extrapolation polynomiale, spline...

FIG. 1

FIG. 2

μNi(μm⁻¹)

FIG. 3

E(eV)

μZr(μm⁻¹)

FIG. 4

E(eV)

FIG. 5

FIG. 6

FIG. 7A

FIG. 7B

FIG. 8A

FIG. 8B

FIG. 9

FIG. 10

FIG. 11

FIG. 12

FIG. 13

Europäisches
Patentamt

European
Patent Office

Office européen
des brevets

**RAPPORT DE RECHERCHE EUROPEENNE**

Numéro de la demande

EP 15 18 5175

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | Manuel Meilinger: "Metal Artifact Reduction and Image Processing of Cone-Beam Computed Tomography Data for Mobile C-Arm CT Devices", Dissertationsreihe Physik - Band 20, 9 juin 2011 (2011-06-09), XP055173216, ISBN: 978-3-86-845074-3 Extrait de l'Internet: URL:http://epub.uni-regensburg.de/20832/1/meilinger.pdf [extrait le 2015-03-03] * pages 41-79 * * pages 26-30 * ----- | 1-10 | INV. G01N23/04 A61B6/00 G06T11/00 |

**DOMAINES TECHNIQUES RECHERCHES (IPC)**

G01N
A61B
G06T

Le présent rapport a été établi pour toutes les revendications

| Lieu de la recherche | Date d'achèvement de la recherche | Examinateur |
|---|---|---|
| La Haye | 17 décembre 2015 | Marzocchi, Olaf |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **J. BAHNART.** *Advanced Tomographic Methods in Materials Reasearch and engineering,* 2008, 145-149

- **VAN DE CASTEELE et al.** A model-based correction method for beam hardening in X-ray microtomography. *J X-ray Sci Technol,* 2004, vol. 12 (1), 43-57